# EUROPEAN PATENT SPECIFICATION

(11) **EP 1 173 060 B1**
(45) Date of publication and mention of the grant of the patent: **21.05.2003**
(21) Application number: 00921263.0
(22) Date of filing: 28.03.2000
(51) Int. Cl.: A01N 35/02, A01N 43/90, A01N 59/00, A61L 2/16, A61L 2/20

(54) **A PROCESS FOR STERILIZING A BIOLOGICALLY CONTAMINATED ENCLOSURE**
VERFAHREN ZUM STERILISIEREN EINES BIOLOGISCH KONTAMINIERTEN GEHÄUSES
METHODE DE STERILISATION D'UNE ENCEINTE CONTAMINEE AU PLAN BIOLOGIQUE

(30) Priority: 29.03.1999 SE 9901137
(43) Date of publication of application: 23.01.2002
(73) Proprietor: Pharma Swede Lund AB, 223 63 Lund (SE)
(72) Inventor: RAHMAN, Shoaa, Abdul, S-224 76 Lund (SE); KITTANEH, Raáfat, Amman (JO)
(74) Representative: Bjerre, Nils B.J.
(86) International application number: SE0000597
(87) International publication number: WO00057700

(56) References cited:
- EP-A1- 0 329 556
- WO-A1-95/22309
- WO-A1-97/23247
- US-A- 4 356 179
- DATABASE CAPLUS [Online] STN INTERNATIONAL DN 103:92906 INTREPRINDEREA DE INDUSTRIALIZARE A CARNII: 'Disinfection of technological spaces', XP002949884 Retrieved from STN Database accession no. 1985:492906 & RO 85 306 B 29 September 1984
- DATABASE CAPLUS [Online] STN INTERNATIONAL DN 80:41064 DENTSU CENTRAL CO., LTD.: 'Telephone disinfectants', XP002949883 Retrieved from STN Database accession no. 1974:41064 & JP 47 045 093 B 14 November 1972

## Description

The present invention relates to a process for sterilizing a biologically contaminated enclosure and particularly to a safe and easy chemical process for a well-controlled release or fumigation of formaldehyde gas from formalin. The process is intended for sterilizing biologically contaminated enclosures such as animal houses, hatcheries, feed stores, feed bins, feed tanks, feed mills, hospitals, medical instruments or other hard to reach areas in which the slowly and well controlled release of formaldehyde gas from easily available formalin solution is responsible for the complete sterilization by maximizing the excellent killing effects against bacteria, viruses and fungi.

### BACKGROUND OF THE INVENTION

### Discussion of related art

It is well known that formaldehyde gas is the mostly used gas in the sterilization of livestock buildings. It is an effective disinfectant against vegetative bacteria, fungi, spores and viruses if an adequate time of exposure and not less than 70% humidity are provided (Remington page 1171, Cooper & Mason 1964).

It can be easily absorbed by surfaces in both its gaseous or liquid state, as it has strong penetration power which increases in completely closed spaces. In a study (Sykes, 1972) it was proved that formaldehyde can attain complete sterilization to a well closed room within two hours at 70% relative humidity.

Its mode of action on living cells is by reaction with the cellular protein and (DNA, RNA) amino acids (Russel, 1976).

Formalin, as a commercially available product, is an aqueous solution containing up to 37% by weight of formaldehyde, HCHO (30.03), with methanol added to prevent polymerization. The solution is extensively used for disinfecting rooms, which have been subjected to infection, by:
1. Reaction of formalin with half its weight of potassium permanganate.
2. Spraying it on sheets hung in the room.
3. Releasing formaldehyde vapor from formalin into the room by a heat generator or heater.
4. Subliming paraformaldehyde powder at 218°C using a heater.

There are disclosed in WO 97/23247 A1 antiperspirant pads which are impregnated with hexamethylenetetramine in an acidic or neutral medium for releasing formaldehyde.

From STN International, File Caplus, Accession no. 1985:492906, Document no. 103:92906, RO 85306 B, it is known to disinfect technological spaces by using formaldehyde which is rapidly released from a formalin solution by a strong exothermic reaction due to the addition of KMnO₄.

In US patent no. 4356179 formaldehyde products are used as agricultural fungicides. The products can for example be obtained by adding hexamethylenetetramine to formaldehyde.

All the above mentioned methods have many disadvantages. Using potassium permanganate to evaporate formaldehyde results in a very vigorous and dangerous reaction, which is completed within few seconds, and this does not give enough time for the worker to escape safely and many fatal accidents have been reported in real life.

It can be proved that spraying formalin on sheets is non-reproducible and does not release enough formaldehyde gas as it is temperature-dependent as can be seen from the following table

| Concentration and type of formaldehyde | Time needed to kill spores | Time needed to kill bacteria |
|---|---|---|
| | | |
| Formaldehyde gas | 2 hours | 2 hours |
| 8% formalin | 18 hours | 18 hours |
| 0.5% formalin | 2-4 days | 6-12 hours |

Formaldehyde gas is thus described as being a sterilizing agent, while formalin (10% strength) is described as being an antiseptic agent; hence there is a need to use the formaldehyde gas as a sterilizer rather than using the formalin, as formalin is time consuming and requires diligent application through decontamination.
Reference: Remington's Pharmaceutical Sciences, 18th. Ed., pages 1171, 1470. Merck Index 11th Ed.

Further, contaminated, highly sophisticated electronic and dental equipment may be damaged by sterilizing liquid agents like those described in US Patent 31779 or by heat or autoclaving etc. US Patent 5552112 introduced a new method for sterilizing a metallic surgical instrument with microwave radiation but the method suffers from non-availability of microwave source to everyone and high cost. As mentioned in US Patent 5552112, gas sterilization with an ethylene oxide mixture is acceptable for both hanpieces and burs. However, this is impractical because of cost of equipment, long sterilization and aeration times involved, and cost of providing adequate protection for personnel. Alkaline glutaraldehyde (2%) as mentioned by Boucher in US Patent 3912450, is used to sterilize equipment, but it must be used for 10 hours to kill spore-forming organisms or tuberculosis microorganisms and is irritating to tissue.

Many workers could successfully release formaldehyde gas using generators or heaters and some patents have been published like US Patents: 4585624, 665794, 1837264, 2993832, 3694146, 3816074, 3898038 and 4166087. All these patents describe different apparatus systems for vaporizing formaldehyde and dispensing it into the enclosure to be sterilized for contact with contaminants. However, such methods suffer from high cost of instruments, maintenance, availability, complexity and restricted volume of formalin to be evaporated. No work has ever been known to use a safe chemical process for the evaporation of formaldehyde as an alternative for the unsafe usage of potassium permanganate or other conventional methods.

### SUMMARY OF THE INVENTION

The present invention provides an ideal, chemical and safe process for releasing formaldehyde gas from a formalin solution by evaporation using heat generated by an exothermic reaction in order to simplify and optimize the use of formaldehyde gas in sterilizing closed spaces, like houses, buildings for housing animals, hospitals, operating rooms, stores, hotels, bath rooms or any object needing to be sterilized.

It is therefore an object of the present invention to provide a process for sterilizing enclosures, such as animal houses, hatcheries, feed stores, feed bins, feed tanks, feed hauling truck bins or tanks, feed mills or other hard to reach areas, or rooms in hospitals, clinics, research laboratories and the like by chemically releasing gaseous formaldehyde into the enclosure for contact with contaminants.

The present invention is based on the finding that the release of formaldehyde gas can be easily provided by heat generated by an exothermic reaction occurring in the presence of formalin. The released formaldehyde gas can be used for sterilizing closed spaces of the kind exemplified above. Exothermic chemical reactions involving various reagents are known to one skilled in the chemical art. In general, a chemist can chose any reagents for such exothermic reactions. Needless to say, it is advantageous to chose reagents, which are easily available and cheep. Once chosen the amounts of reagents sufficient to generate heat for releasing gaseous formaldehyde for sterilization from formalin are easily determined by one skilled in the art. As an example of appropriate reagents there can be mentioned hexamethylenetetramine and peroxide compounds or precursors of peroxide compounds, which when mixed, and in the presence of formalin, bring about an exothermic reaction releasing formaldehyde gas for sterilizing.

According to the invention the exothermic reaction is provided by addition of reagents to the formalin in amounts sufficient to generate heat for releasing formaldehyde from the formalin. In accordance therewith, the reagents comprise a first reagent A and a second reagent B, said reagent A comprising hexamethylenetetramine, optionally in admixture with sulphur sublime, red iron oxide, silica, preferably that sold under the tradename Aerosil, and citric acid, and the second reagent B comprising a peroxide compound, such as hydrogen peroxide, or a precursor thereof.

According to a most preferred embodiment, the reagent A comprises Methenamine (hexamethylenetetramine) and the reagent B comprises a hydrogen peroxide solution. Mostly preferred, said solution contains 10-50% hydrogen peroxide.

In accordance with the present invention the formalin, for example having a concentration of 10-40% formaldehyde gas, is mixed with the reagent A comprising hexamethylenetetramine and the reagent B, for example hydrogen peroxide. As explained below, the temperature of the reaction solution will increase spontaneously by the exothermic chemical reaction and the production of formaldehyde gas starts effectively at 60°C. The temperature rises and release of formaldehyde gas reaches the maximum value at 90°C.

As non-limiting examples of peroxide compounds the following can be mentioned: ammonium peroxosulfate, potassium peroxodisulfate, hydrogen peroxide, acetyl peroxide, benzoyl peroxide and cumene hydroperoxide.

According to a most preferred embodiment, wherein said reagents comprise hexamethylenetetramine and hydrogen peroxide the ratio between these is within the range of 0.7-1.5.

### DETAILED DESCRIPTION OF THE INVENTION:

The present invention is illustrated by the following example:

To evaporate 10 ml up to maximum 3500 ml (preferably 2000 ml) of formalin containing 10%-40% of formaldehyde gas in water:

The above formalin quantity is mixed with 100g-400g, preferably 200 g powder (A) which consists of five ingredients as follows:

| Ingredient | Range in grams | Preferably in grams |
|---|---|---|
| | | |
| Sulphur sublime | 0-10 | 0.30 |
| Iron oxide, red | 0-10 | 0.30 |
| "Aerosil" | 0-5 | 0.40 |
| Citric acid | 0-5 | 4.00 |
| Methenamine | 180-210 | 195.00 |

In case of sterilizing highly sophisticated medical or dental instruments or other similar objects Methenamine (hexamethylenetetramine) without the rest of chemicals shown in the above table should be used.

After mixing the above quantity of powder (A) with the above amount of formalin, (100ml-400ml, best results with 200ml) liquid (B) is added.

It is the powder part, which controls the reaction, and provides enough time for the operator before the onset of the evaporation process.

Liquid (B) is hydrogen peroxide having a concentration of 10%-50% (best results with 50%). It is the heat generated by mixing powder (A) and liquid (B) which helps in heating and evaporation or controlled fumigation of formalin.

After about five minutes, the temperature of the solution increases spontaneously by the exothermic chemical reaction and the production of formaldehyde gas starts effectively at 60° C. With the rise of the temperature of the exothermic reaction the evaporation of formaldehyde gas becomes very strong and reaches the maximum at 90° C (after about 10 minutes).

### EXAMPLES:

### Example 1:

In order to illustrate the invention the following non-limiting example is given:

A 4000 m³ (10000 bird capacity) chicken house was chosen to be sterilized immediately after the complete evacuation of the building and before the new bird cycle. The house was left as such without further cleaning. Swabs were taken from different representative areas of the house (ceiling, flour etc.). All windows and openings were tightly closed. The house was humidified with water to obtain at least 70% relative humidity. The chosen formalin amount for the experiment was 2 l per 100m³, so 40 l of (37%) formaldehyde solution were divided into 20 plastic containers (10 l capacity each) such that each container contains 2 l of formalin. The containers were placed evenly in the house. To each container 200 g of powder (A) were added and consisting of:

| Ingredient | Quantity (g) |
|---|---|
| Sulfur Sublime | 0.30 |
| Iron oxide, red | 0.30 |
| "Aerosil" | 0.40 |
| Citric acid | 4.00 |
| Methenamine | 195.00 |
| TOTAL | 200.00 |

Powder (A) was mixed thoroughly with formalin and thereafter 200 ml of liquid (B) (50% hydrogen peroxide) were added to every container and mixed.

In every container solution temperatures were recorded against time, and the following average solution temperatures were obtained against time.

| Time in minutes | Solution Temperature °C |
|---|---|
| | |
| 3 | 40 |
| 5 | 60 |
| 7 | 80 |
| 10 | 95 |
| 30 | 95 |
| 40 | 85 |
| 50 | 75 |
| 60 | 65 |
| 100 | 40 |

Formaldehyde started evaporating effectively when the temperature of the solution reached 60° C after 5 minutes of mixing all ingredients together.

After 10 minutes the evaporation became extensive (boiling).

This temperature was attained for 20 minutes before it declined and reached room temperature after two hours.

The chicken house was kept closed and empty for 48 hours after which it was well ventilated and swabs were taken again randomly and analyzed for total fungal and bacterial count.

| Average count before sterilization | Average count after sterilization |
|---|---|
| | |
| 1000,000 colonies/g | 2 colonies/g |

### Example 2:

Calculate the size of the house and then put in separate containers (20 liters capacity) two liters of Formalin for each 100 cubic metre. Add 200 g of reagent A by mixing with the standard Formalin in all containers; steer the mix after adding the powder until it is completely dissolved, then add 200 ml of Reagent B to the mixture and leave the house. After five minutes evaporation starts and the release of formaldehyde gas continue for more than four hours and it is preferable to keep the house closed overnight. The production of formaldehyde gas starts effectively at 60°C. The evaporation becomes very strong and reaches the maximum at 90°C after 10 minutes. Fumigation works best at temperatures above 18°C, therefore the house temperature should be maintained above this level and the humidity should be around 70%. The composition of reagent A is sulphur, iron oxide, dicalcium phosphate and hexamethylenetetramine. Reagent A controls the formaline evaporation reaction and provides a suitable time before the onset of evaporation. Reagent B is a liquid comprising 50% hydrogen peroxide which is sufficient to evaporate two litres of 37% formalin solution. Reagent B assists in heating and functions as a catalyst for evaporation of formalin.

Reagent A is to be kept in a cool place at a temperature below 30°C, out of reach of children and feed stock places. Reagent B is also to be kept in a cool place at a temperature below 30°C, out of reach of children and feed stock places.

### Example 3:

The procedure of this example was carried out in the same manner as in example 2 with the exception of that the composition of reagent A is 100% hexamethylenetetramine powder. The results of this experiment were also excellent as in the aforementioned experiments.

The present invention, therefore, is well suited and adapted to attain the intended objects and has the advantages and features mentioned.

### REFERENCES

1. Russell, A.D. 1976 . Inactivation of non-sporing bacteria by gases. SOC.APPL.Bacterial., 5, 61-68.
2. Cooper, E.A., and Mason, J. 1964. Studies of selective bactericidal action. J.HYG, 26, 118-126.
3. Sykes, M.K. 1972 . Sterilization of ventilators. Int. Anesthesiol. Clin., 10, 131.
4. Pharmaceutical CODEX. 1976.
5. Martindale, 1989.
6. Remington, 1990.
7. Disinfection, Sterilization and preservation 1983 third edition, Seymor S. Block.
8. Poultry Production. Sunil Kumar Das, 1994 First edition.

## Claims

1. A process for sterilizing a biologically contaminated enclosure in which the enclosure is brought into contact with formaldehyde, said formaldehyde being released from formalin by heat generated by an exothermic reaction occurring in the presence of said formalin, the exothermic reaction being provided by addition of at least one reagent to said formalin in amounts sufficient to generate heat for releasing formaldehyde from said formalin, **characterized in that** said at least one reagent comprises a first reagent (A) and a second reagent (B), said reagent (A) comprising hexamethylenetetramine, optionally in admixture with sulphur sublime, red iron oxide, silica and citric acid, and said second reagent (B) comprising a peroxide compound or a precursor thereof.

2. A process of claim 1, wherein said reagent (B) comprises a hydrogen peroxide solution.

3. A process of any one of claims 1-2, wherein to evaporate formaldehyde gas from every 10 ml up to maximum 3500 ml of formalin the following ranges of constituents of reagent (A) are needed: (0-10) grams of sulphur sublime, (0-10) grams of red iron oxide, (0-5) grams of silica, (0-5) grams of citric acid and (180-210) grams of hexamethylenetetramine.

4. A process of claim 2, wherein the solution contains 10-50% hydrogen peroxide.

5. A process of any one of claims 1-4, wherein the ratio between the reagents (A, B) is 0.7-1.5.

6. A process of any one of claims 1-5, wherein the formalin has a concentration of 10-40% formaldehyde gas.

## Patentansprüche

1. Verfahren zum Sterilisieren eines biologisch kontaminierten umschlossenen Raumes, in welchem der umschlossene Raum in Kontakt mit Formaldehyd gebracht wird, wobei der Formaldehyd aus Formalin durch Wärme freigesetzt wird, die durch eine exotherme Reaktion erzeugt wird, die in Gegenwart des Formalins erfolgt, wobei die exotherme Reaktion durch Zugabe von mindestens einem Reagenz zu dem Formalin in Mengen herbeigeführt wird, die zum Erzeugen von Wärme zum Freisetzen von Formaldehyd aus dem Formalin ausreichen, **dadurch gekennzeichnet, dass** das mindestens eine Reagenz ein erstes Reagenz (A) und ein zweites Reagenz (B) umfasst, wobei das Reagenz (A) Hexamethylentetramin, gegebenenfalls in Mischung mit Schwefelblüte, rotem Eisenoxid, Siliciumdioxid und Citronensäure, umfasst, und das zweite Reagenz (B) eine Peroxidverbindung oder eine Vorstufe davon umfasst.

2. Verfahren nach Anspruch 1, wobei das Reagenz (B) eine Wasserstoffperoxidlösung umfasst.

3. Verfahren nach einem der Ansprüche 1-2, wobei zum Verdampfen von Formaldehydgas aus jeweils 10 ml bis zu maximal 3500 ml Formalin die folgenden Bereiche der Bestandteile von Reagenz (A) benötigt werden: (0-10) Gramm Schwefelblüte, (0-10) Gramm rotes Eisenoxid, (0-5) Gramm Siliciumdioxid, (0-5) Gramm Citronensäure und (180-210) Gramm Hexamethylentetramin.

4. Verfahren nach Anspruch 2, wobei die Lösung 10-50 % Wasserstoffperoxid enthält.

5. Verfahren nach einem der Ansprüche 1-4, wobei das Verhältnis zwischen den Reagenzien (A, B) 0,7-1,5 beträgt.

6. Verfahren nach einem der Ansprüche 1-5, wobei das Formalin eine Konzentration von 10-40 % Formaldehydgas aufweist.

## Revendications

1. Procédé pour la stérilisation d'une enceinte biologiquement contaminée, dans lequel l'enceinte est mise en contact avec du formaldéhyde, ledit formaldéhyde étant libéré par une solution aqueuse de formaldéhyde par la chaleur générée par une réaction exothermique se produisant en présence de ladite solution aqueuse de formaldéhyde, la réaction exothermique étant fournie par l'addition d'au moins un réactif à ladite solution aqueuse de formaldéhyde en quantités suffisantes pour générer de la chaleur pour libérer le formaldéhyde de ladite solution aqueuse de formaldéhyde, **caractérisé en ce que** ledit au moins un réactif comprend un premier réactif (A) et un second réactif (B), ledit réactif (A) comprenant de l'hexaméthylènetétramine, de façon optionnelle en mélange avec du soufre sublimé, de l'oxyde rouge de fer, de la silice et de l'acide citrique, et ledit second réactif (B) comprenant un composé peroxyde ou un précurseur de celui-ci.

2. Procédé selon la revendication 1, dans lequel ledit réactif (B) comprend une solution de peroxyde d'hydrogène.

3. Procédé selon l'une quelconque des revendications 1 ou 2, dans lequel, pour faire évaporer du formaldéhyde gazeux à partir d'une quantité de 10 ml jusqu'à 3500 ml au maximum de solution aqueuse de formaldéhyde, il faut utiliser les intervalles suivants de constituants de réactif (A) : (0 à 10) grammes de soufre sublimé, (0 à 10) grammes d'oxyde rouge de fer, (0 à 5) grammes de silice, (0 à 5) grammes d'acide citrique et (180 à 210) grammes d'hexaméthylènetétramine.

4. Procédé selon la revendication 2, dans lequel la solution contient 10 à 50 % de peroxyde d'hydrogène.

5. Procédé selon l'une quelconque des revendications 1 à 4, dans lequel le rapport entre les réactifs (A, B) est de 0,7 à 1,5.

6. Procédé selon l'une quelconque des revendications 1 à 5, dans lequel la solution aqueuse de formaldéhyde a une concentration de formaldéhyde gazeux de 10 à 40 %.
